**Europäisches Patentamt**

**(19) European Patent Office**

**Office européen des brevets**

(11) Publication number : **0 386 688 B1**

# (12) EUROPEAN PATENT SPECIFICATION

(45) Date of publication of patent specification :
**21.04.93 Bulletin 93/16**

(51) Int. Cl.$^5$ : **A61K 47/32**

(21) Application number : **90104245.7**

(22) Date of filing : **06.03.90**

(54) Method for preparing hydrogel preparations and xerogel preparations.

(30) Priority : **08.03.89 JP 53779/89**
**12.09.89 JP 234759/89**

(43) Date of publication of application :
**12.09.90 Bulletin 90/37**

(45) Publication of the grant of the patent :
**21.04.93 Bulletin 93/16**

(84) Designated Contracting States :
**AT BE CH DE DK ES FR GB GR IT LI LU NL SE**

(56) References cited :
**FR-A- 2 355 510**
**US-A- 4 525 348**
**STN INTERNATIONAL FILE SAVER**
**(KARLSRUHE) & CHEMICAL ABSTRACTS, vol.**
**110, abstract no. 219086, Columbus, Ohio, US;**
**& JP-A-63 130 540 (KOKAI TOKKYO KOHO)**
**18-11-1986**
**Römpp Chemie Lexicon, 9 Auflage, vol. 1, p.**
**585 and vol. 2, page 1511**

(73) Proprietor : **HOECHST JAPAN LIMITED**
**10-16, 8-chome, Akasaka, Minato-ku**
**Tokyo (JP)**

(72) Inventor : **Goto, Shigeru, Prof.**
**Oazadoi 888-34**
**Higashi-ku, Fukuoka (JP)**
Inventor : **Kawata, Masakazu, Dr.**
**Yamatomachi 1-4-2**
**Kasuga, Fukuoka (JP)**

(74) Representative : **Becker, Heinrich Karl**
**Engelbert, Dr. et al**
**HOECHST AKTIENGESELLSCHAFT Central**
**Patent Department P.O. Box 80 03 20**
**W-6230 Frankfurt am Main 80 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 386 688 B1

## Description

This invention relates to a method for preparing hydrogel preparations and xerogel preparations containing a medicinal component such as pentoxifylline, a cerebral microcirculation-improving agent, or propentofylline, a cerebral nerve cell-protecting and function-improving agent.

In recent years hydrogels have widely been investigated as a medical material because of their high affinity to organic tissues. Particularly, those in which agar, carragenan, collagen, gelatin, alginic acid, polyvinyl alcohol or the like are used are well known as medicinal component-containing hydrogel preparations.

Although these hydrogel preparations have an advantage in that they give little damage to organic tissues, they are disadvantageous in that they have high water content which unfavorably affect mechanical strength and greatly limit their application. Consequently, most of the hydrogel preparations are directed to application to skin, and the presently available preparations are very rarely directed to oral administration, implanting or rectal administration. Extensive studies have recently been made for longer duration of the blood level and efficacy of drugs using a sustained-releasing preparation and attention has been called thereto. However, there are almost no hydrogel preparations found to be succesful in achieving sustained release.

British Patent Specification 1 552 521 is directed to pharmaceutical gels prepared by mixing an aqueous solution of a carboxyvinyl polymer, a water-soluble basic compound and a drug. The basic compound is used for neutralizing the carboxyvinyl polymer, which is water-soluble. No water-insoluble polymer is used in the described process.

With an object of improving the properties of the prior art hydrogel preparations and enhancing functional capability and applicability, we have now invented an unprecedented and entirely new type of hydrogel preparations and xerogel preparations prepared by freeze-drying the former. Paying particular attention to the therapeutic agent for senile dementia which appears to therapeutically play an important role in an aged society, investigations have been made in particular on hydrogel preparations and xerogel preparations containing such therapeutic agent, and finally sustained-releasing preparations for rectal administration which are much better bioavailable than prior-art suppositories have been invented.

The present invention relates to a novel method for preparing hydrogel preparations which comprises suspending an acrylic copolymer in an aqueous solution, suspension or emulsion of a medicinal component such as pentoxifylline or propentofylline in advance dissolved, suspended or emulsified, then adding thereto an aqueous alkaline solution and uniformly stirring the mixture.

Furthermore, the invention is concerned with a novel method for preparing xerogel preparations which comprises freezing the hydrogel preparation thus obtained at a temperature of -10°C below and drying in vacuo the frozen mass.

The acrylic copolymer used in the method of the invention is desirably a methacrylic acid-methyl methacrylate copolymer. As examples of the copolymer are mentioned ®Eudragit L (methacrylate copolymer L (copolymer of methacrylic acid : methyl methacrylate = 1:1)), Eudragit S (methacrylate copolymer S (copolymer of methacrylic acid : methyl methacrylate = 1:2)), ®Eudispert hv (copolymer of methacrylic acid : methyl methacrylate = 2:1, high-viscosity type), Eudispert mv (copolymer of methacrylic acid : methyl methacrylate = 2:1, medium-viscosity type) and Eudispert nv (copolymer of methacrylic acid : methyl methacrylate = 2:1, low-viscosity type).

As the aqueous alkaline solution is mentioned an aqueous solution of sodium hydroxide, potassium hydroxide, calcium hydroxide, ethylenediamine or triethanolamine. Among them, sodium hydroxide is especially desirable.

The amount of sodium or potassium hydroxide to be incorporated was carefully investigated considering the degree of neutralization of the methacrylic acid-methyl methacrylate copolymer with the aqueous alkaline solution and the pH of the hydrogel preparation to be formed. The hydrogels have preferably a pH value of smaller than weakly alkaline. The range between 3.0 and 5.75 mmol per gram of Eudragit L, between 3.0 and 3.5 mmol per gram of Eudragit S, or between 4.0 and 7.5 mmol per gram of Eudispert hv, mv or nv is desirable. If a less or no amount is incorporated, there will be produced no hydrogel preparation at all. If a more amount is incorporated, the hydrogel preparation will become weakly or strongly alkaline and not be applicable to living body.

In addition to the cerebral microcirculation-improving agent pentoxifylline and the cerebral nerve cellprotecting and function-improving agent propentofylline which are preferably incorporated in hydrogel preparations, a variety of medicinal components can be used, for example, non-steroidal antiinflammatory and analgesic agents, anticancer agents, hypotensives, antiarrhythmic agents, antiallergic agents, antihistaminic agents, psychotropic agents, diuretics, bronchodillators and other systemic medicinal agents and local anesthetic agents such as procaine hydrochloride and lidocaine hydrochloride.

Particularly preferable medicinal components are pentoxifylline, propentofylline, furosemide, piretanide, ketoprofen, ibuprofen, naproxen, flurbiprofen, diclofenac sodium, loxoprofen sodium, fenbufen, alclofenac, indomethacin, piroxicam, mefenamic acid, propranolol, procaine hydrochloride, lidocaine hydroxide, theophylline, peptides, for example buserelin.

The novel preparative method of the invention will be described in particular below.

First, a medicinal component (for example, pentoxifylline) is dissolved, suspended or emulsified in water, to which is then added an acrylic copolymer such as a methacrylic acid-methyl methacrylate copolymer (for example, Eudispert hv) in a range of 1-20 % (w/w) of the total weight. The mixture is mechanically stirred and allowed to soak and swell for 10-15 min. To the resulting mixture is added with stirring 4.0-7.5 mmol of an aqueous alkaline solution (for example, an aqueous solution of sodium hydroxide). Stirring is terminated immediately when coagulated. The product is an opaque coagulated mass at this stage but becomes a transparent uniform hydrogel preparation as gelation progresses. In order to promote the gelation heating at 50-60°C may be applied. According to the preparation method of the invention hydrogel preparations in various forms including solution, gel, jelly and sponge can be prepared. If the mixture of a medical component and an acrylic copolymer are filled in an appropriate forming vessel before coagulation but after adding an aqueous alkaline solution with stirring, there can be prepared hydrogel preparations with the desired form maintained, for example the form of a spindle. Thus, because of easy control of the release of the medicinal component it is possible to prepare a wide variety of hydrogel preparations from prompt releasing to sustained releasing of the active substance.

In preparing a xerogel preparation the hydrogel preparation obtained above is degassed by appropriate means (for example, centrifugation, ultrasonication), then filled in an appropriate forming vessel (for example, a plastic mold for the formation of suppositories or a polypropylene syringe cut to an appropriate length) and frozen by cooling at a temperature of -10°C or below (desirably around -70 to -80°C). The hydrogel thus frozen is rapidly transferred into a freezing flask which has been cooled to around the above-mentioned temperature and immediately subjected to vacuum drying for a predetermined period of time. Then, there can be prepared a xerogel preparation with the desired form maintaned. In this operation external cooling may be applied to increase drying efficiency. As the coolant there may be employed dry ice-acetone, dry ice-methanol or liquid nitrogen as needed. If formed while maintaining a geometrical form such as spindle shape, cylinder or sphere, it is necessary to set the vacuum drying time in such a manner that the water content is not completely eliminated (for example, 3-5 % of the water content remains uneliminated). Thus, it is a distinct characteristic of the invention to allow a very small amount of water remained to act as binder between the polymer chains so that the form of the xerogel is kept unchanged. Accordingly, the vacuum drying time is set at about 6-8 hours when a freeze-drying vessel used on a laboratory scale is employed, though it is variable depending upon grade of the instrument used. On the other hand, if a powdery xerogel after complete drying is to be produced, an intact hydrogel is subjected to the above-described procedures in a freezing flask to attain freeze drying to a constant weight without advance transfer of the hydrogel to a forming vessel. Thus, according to the invention, the xerogels formed into spindles or cylinders can be employed mainly, as a preparation for rectal administration, and the powdery xerogels can be employed as a preparation for oral administration or implanting after graded and filled into capsules, or mechanically compressed and formed by means, for example, of a tabletting machine to prepare a matrix preparation. Especially, the xerogel preparations designed for rectal administration can easily be applied to patients having difficulties in oral administration such as children and aged persons, and have the merit of letting the medicinal component (for example, pentoxifylline and propentofylline), which might be reduced in its efficacy by the first passing through liver after oral administration, pass from rectum directly into circulation.

The invention will be described in more detail below with reference to Examples, which, however, are not intended to limit the invention.

**Example 1**

1.5 g of Eudispert hv is added to 5.0 g of a 5 % aqueous solution of pentoxifylline. The mixture is stirred for 1 min and then allowed to stand for 10 min at room temperature. To the resulting mass there are added, with stirring, 3.5 ml of a 3N aqueous solution of sodium hydroxide. The stirring is terminated immediately before coagulation occurs.

The reaction mixture is filled in plastic molds each contaning 2 g of the mixture and allowed to stand until gelation is completed. There is produced a clear, homogeneous hydrogel preparation in spindles containing 2.5 % pentoxifylline designated for rectal administration.

Many kinds of administration forms can be prepared according to the method described.

### Example 2

To 0,25 g of furosemide there are added 4.75 g of purified water and 1.5 g of Eudispert hv. The mixture is stirred for 10 min and then allowed to stand for 15 min at room temperature. To the resulting mass there are added, with stirring, 3.5 ml of a 3N aqueous solution of sodium hydroxide. The stirring is terminated when coagulation occurs. The reaction mixture is allowed to stand until gelation is completed. There is produced a white, homogeneous hydrogel preparation containing 2.5 % furosemide.

### Example 3

To 0.25 g of ketoprofen there are added 4.75 g of purified water and 1.5 g of Eudispert hv. The mixture is stirred for 10 min and then allowed to stand for 10 min at room temperature. To the resulting mass there are added with stirring 3.5 ml of a 3N aqueous solution of sodium hydroxide. The stirring is terminated when coagulation occurs. The reaction mixture is allowed to stand until gelation is completed. There is produced a clear, homogeneous hydrogel preparation containing 2.5 % ketoprofen.

### Example 4

2.0 g of Eudragit S are added to 6.5 g of a 3.85 % aqueous solution of pentoxifylline. The mixture is stirred for 1 min and then allowed to stand for 10 min at room temperature. To the resulting mass there are added with stirring 1.5 ml of a 4N aqueous solution of sodium hydroxide. The stirring is terminated when coagulation occurs. The reaction mixture is allowed to stand until gelation is completed. There is produced a clear, homogeneous hydrogel preparation containing 2.5 % pentoxifylline.

### Example 5

3.0 g of Eudragit L are added to 4.5 g of a 5.56 % aqueous solution of pentoxifylline. The mixture is stirred for 1 min and then allowed to stand for 15 min at room temperature. To the resulting mass there are added with stirring 2.5 ml of a 6N aqueous solution of sodium hydroxide. The stirring is terminated when coagulation occurs. The reaction mixture is allowed to stand until gelation is completed. There is produced a clear, homogeneous hydrogel preparation containing 2.5 % pentoxifylline.

### Example 6

To 0.25 g of piretanide there are added 4.75 g of purified water and 1.5 g of Eudispert mv. The mixture is stirred for 15 min and then allowed to stand for 10 min at room temperature. To the resulting mass there is added with stirring 3.5 ml of a 3N aqueous solution of sodium hydroxide. The stirring is terminated when coagulation occurs. The reaction mixture is allowed to stand until gelation is completed. There is produced a yellowish white, homogeneous hydrogel preparation containing 2.5 % piretanide.

### Example 7

1.5 g of Eudispert nv are added to 0.25 g of propentofylline and 4.75 g of purified water. The mixture is stirred for 10 min and then allowed to stand for 15 min. To the resulting mass there are added with stirring 3.5 ml of 3N aqueous solution of sodium hydroxide. The stirring is terminated when coagulation occurs. The reaction mixture is allowed to stand until gelation is completed. There is produced a clear, homogeneous hydrogel preparation containing 2.5 % propentofylline.

### Example 8

1.5 g of Eudispert nv are added to 5.0 g of a 5 % aqueous solution of pentoxifylline. The mixture is stirred at 20°C for 10 min. To the resulting mass there are added with stirring 3.5 ml of a 3N aqueous solution of sodium hydroxide. The stirring is terminated when coagulation occurs. The reaction mixture is allowed to stand at 55°C for 10 min until gelation is completed. There is produced a clear, homogeneous hydrogel preparation containing 2.5 % pentoxifylline.

**Example 9**

1.5 g of Eudispert hv are added to 5 g of a 5 % aqueous solution of pentoxifylline. The mixture is stirred at 20°C for 10 min. To the resulting mixture there are added with stirring 3.5 ml of a 3N aqueous solution of sodium hydroxide. The stirring is terminated when coagulation occurs. The reaction mixture is allowed to stand at 55°C for 10 min until gelation is completed. A clear, homogeneous hydrogel thus obtained is degassed, subsequently filled in plastic molds each containing 2 g of the hydrogel and stored frozen at -10°C for 12 hours. The frozen hydrogel thus obtained is rapidly transferred to a freezing flask cooled to -80°C, which is allowed to stand for 30 min and then promptly subjected to drying in vacuo for 8 hours. There is produced a xerogel preparation in spindles (each containing 50 mg of pnetoxifylline) designed for rectal administration.

**Example 10**

1 g of Eudispert hv is added to 6.5 g of a 3.85 % aqueous solution of pentoxifylline. The mixture is stirred at 20°C for 10 min. To the resulting mixture there are added with stirring 2.5 ml of a 3N aqueous solution of sodium hydroxide. The stirring is terminated when coagulation occurs. The homogeneous hydrogel obtained is degassed and is subsequently filled in polypropylene syringes each containing 2 g of the hydrogel and stored frozen at -10°C for 12 hours. The frozen hydrogel thus obtained is rapidly transferred to a freezing flask cooled to -80°C, which is allowed to stand for 30 min and then promptly subjected to drying in vacuo for 8 hours. There is produced a xerogel preparation in cylinders (each containing 50 mg of pentoxifylline) designed for rectal administration.

**Example 11**

3.0 g of Eudragit L are added to 4.5 g of a 5.56 % aqueous solution of propentofylline. The mixture is stirred for 5 min and allowed to stand for 15 min at room temperature. To the resulting mixture there are added 2.5 ml of a 6N aqueous solution of sodium hydroxide. The homogeneous hydrogel thus obtained is filled in plastic molds each containing 2 g of the hydrogel, frozen at -76°C and then subjected to drying in vacuo for 8 hours. There is produced a xerogel preparation in spindles (each containing 50 mg of propentafylline) designed for rectal administration.

**Example 12**

To 25 g of piretanide there are added 475 ml of purified water and 150 g of Eudispert mv. The mixture is stirred for 10 min followed by addition with stirring of 350 ml of 3N sodium hydroxide solution. Hydrogel thus obtained is dried in vacuo to a constant weight. Xerogel thus produced is pulverized by means of a pulverizer, granules having particle sizes of 250-500 μm are collected and tabletted to obtain matrix tablets each having a weight of approximately 50 mg (each containing 6 mg of piretanide) for oral administration.

**Example 13**

To 50 g of ketoprofen there are added 400 ml of purified water and 200 g of Eudragit S. The mixture is stirred for 10 min followed by addition with stirring of 150 ml of 4N sodium hydroxide solution. The same procedures as in Example 12 are followed. There are obtained matrix tablets each having a weight of approximately 260 mg (each containing 50 mg of ketoprofen) for oral administration.

**Example 14**

To 0.5 g of buserelin acetate there are added 475 ml of purified water and 150 g of Eudispert mv. The mixture is stirred for 15 min followed by addition with stirring of 350 ml of a 3N aqueous solution of sodium hydroxide. Hydrogel thus obtained is dried in vacuo to a constant weight. Xerogel thus produced is pulverized by means of a pulverizer, granules having particle sizes of 250-500 μm are collected and then tabletted to obtain a matrix preparation having a tablet weight of approximately 35 mg (100 μg of buserelin acetate per tablet) for implanting.

5

## Tests

### I Release test

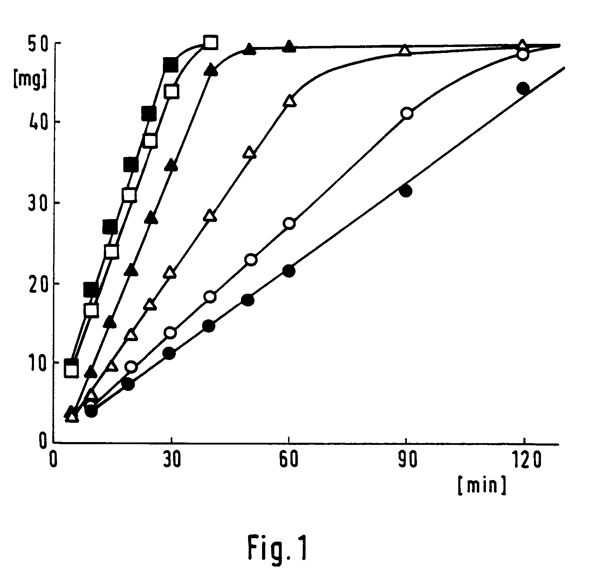

Various hydrogel preparations in spindles containing 2.5 % pentoxifylline designed for rectal administration prepared according to the method of the invention in analogy to Examples 1 to 8 were subjected to a release test (Test method: The rotating disc method; Test conditions: 37°C, pH 6.8). The results are shown in Fig. 1. The curves in Fig. 1 respectively represent a pentoxifylline-releasing (amount of released pentoxifylline in mg) curve for the preparations of Table 1.

## Table 1

| Fig. 1 | Example | NaOH Concent. | Amount | Pentoxifylline Concent. | Amount | Eudispert or Eudragit Amount |
|---|---|---|---|---|---|---|
| ▬ | 4 | 4N | 1.5 ml | 3.85 % | 6.5 g | 2.0 g of Eudragit S |
| ▭ | 5 | 6N | 2.5 ml | 5.56 % | 4.5 g | 1.5 g of Eudragit L |
| ▲ | 8 | 3N | 3.5 ml | 5.00 % | 5.0 g | 1.5 g of Eudispert nv |
| △ | - | 3N | 3.5 ml | 5.00 % | 5.0 g | 1.5 g of Eudispert mv |
| ○ | 1 | 3N | 3.5 ml | 5.00 % | 5.0 g | 1.5 g of Eudispert hv |
| ● | - | 6N | 2.5 ml | 5.56 % | 4.5 g | 3.0 g of Eudispert hv |

### II Absorption test in rabbits

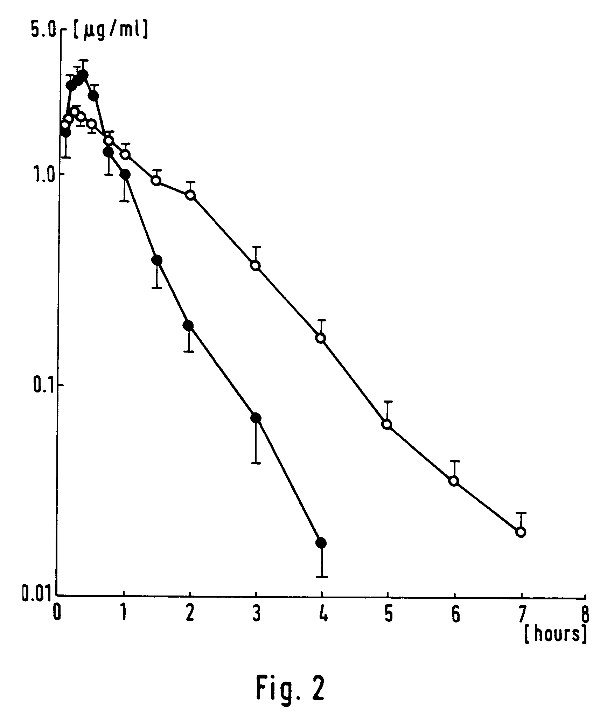

A hydrogel preparation prepared according to the method of the invention and formed by the same method as described in Example 1 in spindle and a control suppository (9.75 g of Witepsol® S-55 was warmed and melted, and 0.25 g of pentoxifylline was added in it. 2 g of the mixture was filled up in each plastic mold and allowed to stand to give a spindle shaped control suppository for rectal administration which contained 50 mg of pentoxifylline) each containing 2.5 % pentoxifylline were rectally administered to rabbits, and plasma levels of the drug were measured. The results are shown in Fig. 2. In Fig. 2,(-○-) represent the plasma pentoxifylline level in μg/ml after administration of an Eudispert hv-hydrogel preparation (4,5 g of a 5,56 % aqueous solution of pentoxifylline, 3,0 g of Eudispert hv, 2,5 ml of 6N NaOH) and -●- represents the plasma pentoxifylline level after the administration of the control suppository respectively.

### III Release test

Various hydrogel or xerogel preparations and a control suppository in spindle shape each containing 50 mg of pnetoxifylline designed for rectal administration were subjected to a drug release test by the Muranishi method.

A release test apparatus for suppository of Muranishi type (Model TMS-1U3; manufactured by Toyama Sangyo) was used.

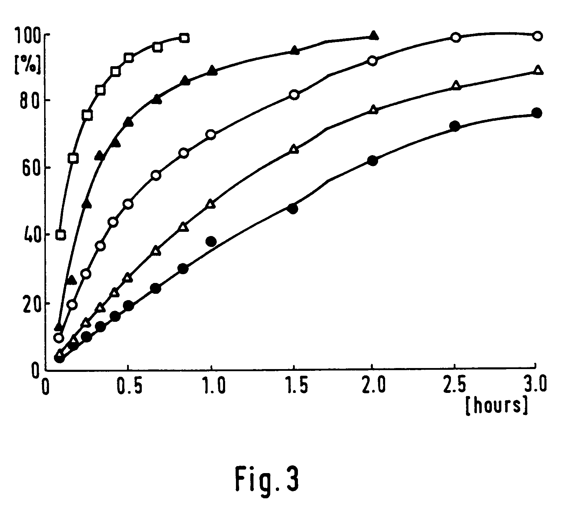

A hydrogel or a xerogel suppository prepared according to the method of invention in analogy to Examples 1 to 14 or Witepsol S-55 suppository (control suppository) each containing 50 mg of pentoxifylline was carefully placed on a screen at the bottom of a cylindrical cell which had been set in a depth of 2 mm from the liquid surface of the release phase (0.2 M $KH_2PO_4$ - 0.2 N NaOH buffer, pH 7.4, 500 ml, 37°C), and immediately thereafter the test was initiated. The release phase and the cell were stirred during the test at a rate of 100 rpm and 10 rpm, respectively. One-milliliter aliquot was taken out at intervals after initiation of the test and replaced by 1 ml of the buffer of the same composition at the same temperature. The collected sample solution, was appropriately diluted and measured for the absorbance at 275 nm. The results (release of pentoxifylline in % over a period of time in hours) are shown in Fig. 3.

The curves in Fig. 3 respectively represent a pentoxifylline-releasing curve for the preparations of Table 2

**Table 2**

| Fig. 3 | Example | NaOH Concent. | Amount | Pentoxifylline Concent. | Amount | Eudispert Amount | Preparation type |
|---|---|---|---|---|---|---|---|
| ▲ | 1 | 3N | 3.5 ml | 5 % | 5.0 g | 1.5 g of Eudispert hv | Hydrogel preparation |
| △ | 9 | 3N | 3.5 ml | 5 % | 5.0 g | 1.5 g of Eudispert hv | Xerogel preparation |
| ○ | 10 | 3N | 2.5 ml | 3.85 % | 6.5 g | 1.0 g of Eudispert hv | Xerogel preparation |
| ● | - | 3N | 3.5 ml | 3.85 % | 6.5 g | 1.0 g of Eudispert hv | Xerogel preparation |

▢ Control Suppository; Witepsol S-55 suppository prepared according to the following method:

9.75 g of Witepsol S-55 was warmed and melted, and 0.25 g of pentoxifylline was added in it. 2 g of the mixture was filled up in each plastic mold and allowed to stand to give a spindles shaped control suppository for rectal administration which contained 50 mg of pentoxifylline.

EP 0 386 688 B1

**IV Absorption test in rabbits**

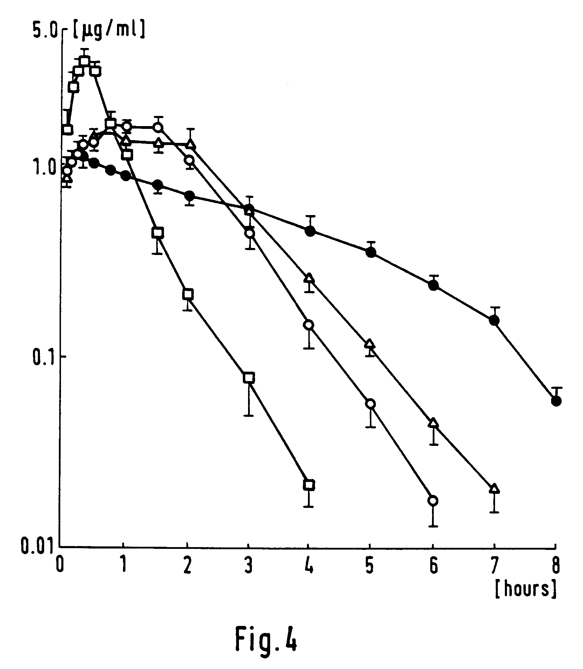

To white male rabbits (weighing 3.0-3.5 kg) fasted for 36 hours were rectally administered various xerogels prepared according to the method of the invention as described in Examples 9 and 10 or a control suppository (Witepsol S-55 suppository) each containing 50 mg of pentoxifylline. Thereafter, 2.5 ml of blood was collected at intervals. The collected blood was immediately centrifuged at 8000 rpm for 5 min. To 1 ml of the plasma thus obtained there were added 0.2 ml of 0.5 mg % aqueous solution of caffeine (internal standard substance) and 5 ml of methylene chloride, and the mixture was shaken for 10 min for extraction. The resulting mixture was centrifuged at 3000 rpm for 10 min, and then 4 ml of the organic layer was collected and evaporated at 45°C to remove the solvent. To the residue there was added 0.2 ml of a moving phase (acetic acid-methanol-water (1:30:70)), and the mixture was thoroughly blended. The blend was then introduced into a high performance liquid chromatography (Model LC-6A, manufactured by Shimazu Seisakusho) for assay of pentoxifylline. There was employed an ODS column (0.6 x 15 cm), and measurement was made at a wavelength of 275 nm. The results (plasma concentration of pentoxifylline in μg/ml) are shown in Fig. 4.

The curves in Fig. 4 represent a plasma concentration-time curve of pentoxifylline respectively for the preparations of Table 3

**Table 3**

| Fig. 4 | Example | NaOH Concent. | Amount | Pentoxifylline Concent. | Amount | Eudispert Amount | Preparation type |
|---|---|---|---|---|---|---|---|
| -○- | 10 | 3N | 2.5 ml | 3.85 % | 6.5 g | 1.0 g of Eudispert hv | Xerogel preparation |
| -●- | - | 2N | 2.5 ml | 3.85 % | 6.5 g | 1.0 g of Eudispert hv | Xerogel preparation |
| -△- | 9 | 3N | 3.5 ml | 5 % | 5.0 g | 1.5 g of Eudispert hv | Xerogel preparation |

-□- Control Suppository; Witepsol S-55 suppository prepared according to the following method;

9.75 g of Witepsol S-55 was warmed and melted, and 0.25 g of pentoxifylline was added in it. 2 g of the mixture was filled up in each plastic mold and allowed to stand to give a spindles shaped control suppository for rectal administration which contained 50 mg of pentoxifylline.

takes place as the acrylic copolymer base is dissolved.

Thus, in a release experiment using an apparatus which allows a solvent to contact with a constant area (for example, the rotating disc method), the drug exhibited a zero order-releasing behavior (see Fig. 1). It is also demonstrated that release of pentoxifylline from the cylinder-form xerogel is more controllable than that from the Witepsol suppository or the hydrogel and that the xerogel is useful as rectal gel preparation of controlled release type (see Fig. 3). The plasma drug concentration-time curve produced when the present hydrogel or xerogel preparation is rectally given to rabbits indicates a favorable pattern of sustained releasing which represents 1.4-fold increase of the relative bioavailability as compared with the control suppository (see Fig. 2 and Fig. 4). Such releasing behaviors are advantageous in maintaining a constant blood level for prolongation of the therapeutic effect. Thus, the present hydrogel preparations are useful for topical or rectal administration as unprecedented and entirely new sustained-release, longlasting preparation of depot-type, and furthermore, expected for a wide range of applications including those for senile dementia patients and pediatric patients.

A variety of medicinal components including those easily inactivated by the first passing through liver can be imbedded in the present hydrogel preparations, which makes the invention highly vauable.

Moreover, it is found that the xerogel preparation which has a firmer structure as compared with the hydrogel preparation is more easily handled for administration.

Thus, the present xerogel preparations are useful for rectal or oral administration as unprecedented and entirely new type of sustained-release preparation and expected for a wide range of applications including those for senile dementia patients and pediatric patients. A variety of medicinal components including those easily inactivated by the first passing through liver can be imbedded in the present xerogel preparations, which makes the invention highly valuable. Furthermore, xerogel preparations of various forms can be produced by controlling the forming operation, nature of the forming apparatus and time for freeze drying, which enable a wide range of applications in the living body. As described above, the preparation method of the present invention is very useful in providing preparations highly valuable in pharmaceutical industry.

Brief Description of the Drawings

Fig. 1 and Fig. 3 represent a pentoxifylline-releasing curve respectively for the hydrogel preparation (by the rotating disc method) and the xerogel preparation (by the Muranishi method). Fig. 2 and Fig. 4 respectively represent a plasma concentration curve of pentoxifylline for the hydrogel preparation and the xerogel preparation.

## Claims

1. A method for preparing hydrogel preparations which comprises suspending an acrylic copolymer in an aqueous solution, suspension or emulsion of a medicinal component, adding to the mixture a predetermined amount of an aqueous alkaline solution and then uniformly stirring the mixture to effect gelation.

2. A method for preparing hydrogel preparations according to Claim 1 characterized by that the acrylic copolymer is a methacrylic acid-methyl methacrylate copolymer.

3. A method for preparing hydrogel preparations according to Claim 1 characterized by that the medicinal component is pentoxifylline, propentofylline, furosemide, piretanide, ketoprofen, ibuprofen, naproxen, flurbiprofen, diclofenac sodium, loxoprofen sodium, fenbufen, alclofenac, indomethacin, piroxicam, mefenamic acid, propranolol, procaine hydrochloride, lidocaine hydroxide, buserelin or a salt thereof or theophylline.

4. A method for preparing hydrogel preparations according to Claim 1 characterized by that the aqueous alkaline solution is an aqueous solution of sodium hydroxide, calcium hydroxide, ammonium hydroxide, ethylene diamine, or triethanol amine.

5. A method for preparing hydrogel preparations according to Claim 1 characterized by that at least one other following conditions are observed.
   a) the medicinal component is pentoxifylline or propentofylline,
   b) the acrylic copolymer is a methacrylic acid-methyl methacrylate copolymer
   c) the alkaline solution is an aqueous solution of sodium hydroxide.

6. A method for preparing xerogel preparations which comprises bringing the hydrogel preparations prepared according to the method of Claims 1 into an appropriate shape by freezing at a temperature of -10°C or below and dehydrating and drying the frozen mass by vacuum drying.

7. A method for preparing xerogel preparations according to Claim 6 characterized by that the acrylic copolymer is a methacrylic acid-methyl methacrylate copolymer.

8. A method for preparing xerogel preparations according to Claim 6 characterized by that the medicinal component is pentoxifylline, propentofylline, furosemide, piretanide, ketoprofen, ibuprofen, naproxen, flurbiprofen, diclofenac sodium, loxoprofen sodium; fenbufen, alclofenac, indomethacin, piroxicam, mefenamic acid, propranolol, procaine hydrochloride, lidocaine hydroxide, buserelin or a salt thereof or theophylline.

9. A method for preparing xerogel preparations according to Claim 6 characterized by that the aqueous alkaline solution is an aqueous solution of sodium hydroxide, calcium hydroxide, ammonium hydroxide, ethylene diamine, or triethanol amine.

10. A method for preparing xerogel preparations according to Claim 6 characterized by that at least one of the following conditions are observed
   a) the medicinal component is pentoxifylline or propentofylline
   b) the acrylic copolymer is a methacrylic acid-methyl methacrylate copolymer
   c) the alkaline solution is an aqueous solution of sodium hydroxide.


## Patentansprüche

1. Verfahren zur Herstellung von Hydrogelpräparaten, welches ein Suspendieren eines Acrylcopolymers in einer wäßrigen Lösung, Suspension oder Emulsion einer medizinischen Komponente, ein Zusetzen einer vorbestimmten Menge einer wäßrigen alkalischen Lösung zu dem Gemisch und dann ein gleichmäßiges Rühren des Gemisches zur Bewirkung der Gelierung umfaßt.

2. Verfahren zur Herstellung von Hydrogelpräparaten nach Anspruch 1, dadurch gekennzeichnet, daß das Acrylcopolymer ein Methacrylsäure-Methylmethacrylat-Copolymer ist.

3. Verfahren zur Herstellung von Hydrogelpräparaten nach Anspruch 1, dadurch gekennzeichnet, daß die medizinische Komponente Pentoxifyllin, Propentofyllin, Furosemid, Piretanid, Ketoprofen, Ibuprofen, Naproxen, Flurbiprofen, Diclofenacnatrium, Loxoprofennatrium, Fenbufen, Alclofenac, Indomethacin, Piroxicam, Mefenaminsäure, Propranolol, Procainhydrochlorid, Lidocainhydroxid, Buserelin oder ein Salz hiervon oder Theophyllin ist.

4. Verfahren zur Herstellung von Hydrogelpräparaten nach Anspruch 1, dadurch gekennzeichnet, daß die wäßrige alkalische Lösung eine wäßrige Lösung von Natriumhydroxid, Calciumhydroxid, Ammoniumhydroxid, Ethylendiamin oder Triethanolamin ist.

5. Verfahren zur Herstellung von Hydrogelpräparaten nach Anspruch 1, dadurch gekennzeichnet, daß wenigstens eine der folgenden Bedingungen eingehalten wird:
   a) die medizinische Komponente ist Pentoxifyllin oder Propentofyllin,
   b) das Acrylcopolymer ist ein Methacrylsäure-Methylmethacrylat-Copolymer,
   c) die alkalische Lösung ist eine wäßrige Lösung von Natriumhydroxid.

6. Verfahren zur Herstellung von Xerogelpräparaten, welches ein Überführen der nach dem Verfahren von Anspruch 1 hergestellten Hydrogelpräparate in eine entsprechende Form durch Gefrieren bei einer Temperatur von -10°C oder darunter und ein Dehydratisieren und Trocknen der gefrorenen Masse durch Vakuumtrocknen umfaßt.

7. Verfahren zur Herstellung von Xerogelpräparaten nach Anspruch 6, dadurch gekennzeichnet, daß das Acrylcopolymer ein Methacrylsäure-Methylmethacrylat-Copolymer ist.

8. Verfahren zur Herstellung von Xerogelpräparaten nach Anspruch 6, dadurch gekennzeichnet, daß die medizinische Komponente Pentoxifyllin, Propentofyllin, Furosemid, Piretanid, Ketoprofen, Ibuprofen, Naproxen, Flurbiprofen, Diclofenacnatrium, Loxoprofennatrium, Fenbufen, Alclofenac, Indomethacin, Piroxicam, Mefenaminsäure, Propranolol, Procainhydrochlorid, Lidocainhydroxid, Buserelin oder ein Salz hiervon oder Theophyllin ist.

9. Verfahren zur Herstellung von Xerogelpräparaten nach Anspruch 6, dadurch gekennzeichnet, daß die wäßrige alkalische Lösung eine wäßrige Lösung von Natriumhydroxid, Calciumhydroxid, Ammoniumhydroxid, Ethylendiamin oder Triethanolamin ist.

10. Verfahren zur Herstellung von Xerogelpräparaten nach Anspruch 6, dadurch gekennzeichnet, daß wenigstens eine der folgenden Bedingungen eingehalten wird:
   a) die medizinische Komponente ist Pentoxifyllin oder Propentofyllin
   b) das Acrylcopolymer ist ein Methacrylsäure-Methylmethacrylat-Copolymer,
   c) die alkalische Lösung ist ein wäßrige Lösung von Natriumhydroxid.

## Revendications

1. Procédé pour préparer des préparations d'hydrogel, qui comprend la mise en suspension d'un copolymère acrylique dans une solution, suspension ou émulsion aqueuse d'un constituant médicinal, l'addition au mélange d'une quantité prédéterminée d'une solution aqueuse alcaline, puis l'agitation uniforme du mélange pour effectuer la gélification.

2. Procédé pour préparer des préparations d'hydrogel selon la revendication 1, caractérisé en ce que le copolymère acrylique est un copolymère acide méthacrylique-méthacrylate de méthyle.

3. Procédé pour préparer des préparations d'hydrogel selon la revendication 1, caractérisé en ce que le constituant médicinal est la pentoxifylline, la propentofylline, le furosémide, le pirétanide, le kétoprofène, l'ibuprofène, le naproxène; le flurbiprofène, le diclofénac sodique, le loxoprofène sodique, le fenbufène, l'alclofénac, l'indométhacine, le piroxicam, l'acide méfénamique, le propranolol, la procaïne chlorhydrate, la lidocaïne hydroxyde, la buséréline ou un de ses sels, ou la théophylline.

4. Procédé pour préparer des préparations d'hydrogel selon la revendication 1, caractérisé en ce que la solution aqueuse alcaline est une solution aqueuse d'hydroxyde de sodium, d'hydroxyde de calcium, d'hydroxyde d'ammonium, d'éthylènediamine ou de triéthanolamine.

5. Procédé pour préparer des préparations d'hydrogel selon la revendication 1, caractérisé en ce que l'une au moins des autres conditions suivantes est satisfaite :
   a) le constituant médicinal est la pentoxifylline ou la propentofylline,
   b) le copolymère acrylique est un copolymère acide méthacrylique-méthacrylate de méthyle,
   c) la solution alcaline est une solution aqueuse d'hydroxyde de sodium.

6. Procédé pour préparer des préparations de xérogel, qui comprend l'opération qui consiste à amener les préparations d'hydrogel préparées selon le procédé de la revendication 1 dans une forme appropriée par congélation à une température inférieure ou égale à -10°C et à déshydrater et à sécher la masse congelée par séchage sous vide.

7. Procédé pour préparer des préparations de xérogel selon la revendication 6, caractérisé en ce que le copolymère acrylique est un copolymère acide méthacrylique-méthacrylate de méthyle.

8. Procédé pour préparer des préparations de xérogel selon la revendication 6, caractérisé en ce que le constituant médicinal est la pentoxifylline, la propentofylline, le furosémide, le pirétanide, le kétoprofène, l'ibuprofène, le naproxène, le flurbiprofène, le diclofénac sodique, le loxoprofène sodique, le fenbufène, l'alclofénac, l'indométhacine, le piroxicam, l'acide méfénamique, le propranolol, la procaïne chlorhydrate, la lidocaïne hydroxyde, la buséréline ou un de ses sels, ou la théophylline.

9. Procédé pour préparer des préparations de xérogel selon la revendication 6, caractérisé en ce que la solution aqueuse alcaline est une solution aqueuse d'hydroxyde de sodium, d'hydroxyde de calcium, d'hydroxyde d'ammonium, d'éthylènediamine ou de triéthanolamine.

**10.** Procédé pour préparer des préparations de xérogel selon la revendication 6, caractérisé en ce que l'une au moins des autres conditions suivantes est satisfaite :

a) le constituant médicinal est la pentoxifylline ou la propentofylline,

b) le copolymère acrylique est un copolymère acide méthacrylique-méthacrylate de méthyle,

c) la solution alcaline est une solution aqueuse d'hydroxyde de sodium.

Fig.1

Fig. 2

Fig. 3

Fig. 4